(19) Europäisches Patentamt
European Patent Office
Office européen des brevets

(11) Veröffentlichungsnummer: **0 059 441**
A1

# (12) EUROPÄISCHE PATENTANMELDUNG

(21) Anmeldenummer: 82101457.8

(22) Anmeldetag: 25.02.82

(51) Int. Cl.³: **A 61 K 31/215**
**A 61 K 31/19, A 61 K 7/48**
**A 61 K 7/06**
**//(A61K31/19, 31/045)**

---

(30) Priorität: 02.03.81 DE 3107895
21.08.81 DE 3133132
15.09.81 DE 3136481

(43) Veröffentlichungstag der Anmeldung:
08.09.82 Patentblatt 82/36

(84) Benannte Vertragsstaaten:
AT BE CH DE FR GB IT LI LU NL SE

(71) Anmelder: Füssener Textil AG

D-8958 Füssen/Ostallgäu(DE)

(72) Erfinder: Stüber, Siegfried, Dr.
Drehergasse 46
D-8958 Füssen/Ostallgäu(DE)

(74) Vertreter: Berg, Wilhelm, Dr. et al,
Dr. Berg, Dipl.-Ing. Stapf, Dipl.-Ing. Schwabe, Dr. Dr.
Sandmair Mauerkircherstrasse 45
D-8000 München 80(DE)

---

(54) Bioaktives Mittel auf Terpen/alpha-Ketocarbonsäure-Basis.

(57) Es hat sich überraschenderweise herausgestellt, daß bioaktive Mittel, wie Arzneimittel, kosmetische Mittel und dergl., welche α-Keto-mono- und/oder di-carbonsäure-terpenylester oder in Kombination Terpene und α-Keto-mono- und/oder dicarbonsäuren, gegebenenfalls in Form ihrer Ester bzw. Salze enthalten, überraschende Wirkungen zeigen. Die Wirkungen entsprechen z.B. denen corticoider Systeme, obwohl strukturell zu solchen Systemen keinerlei chemische Verwandtschaft besteht.

Die Rückbesinnung der jüngeren medizinischen Forschung auf die wertvolle Anwendung natürlicher Kräfte hat dazu geführt, daß zunehmend Arzneimittel und kosmetische Mittel auf natürlicher Grundlage wieder zum Einsatz kommen, da man bei weitem nicht in dem Maß schädliche Nebenwirkungen befürchten muß, wie bei rein synthetischen Substanzen. Terpene wurden schon früher in der Medizin verwendet. So findet z.B. gereinigtes Terpentinöl, das im wesentlichen die Monoterpene $\alpha$-Pinen, $\Delta^3$-Caren, Bornylacetat und Terpinen-4-ol enthält, schon seit langem äußerliche Anwendung in Form von Pflastern, Salben, Linimenten als hautreizendes, hyperämisierendes Mittel bei rheumatischen Beschwerden, Neuralgien, Arthritis, Furunkel. Dabei tritt eine Erhöhung der Durchblutung im Bereich der lokalen Applikation ein und dadurch eine geringe temporäre Linderung der Beschwerden. Lit.: Hildebert Wagner, Pharmazeutische Biologie, 2. Drogen und ihre Inhaltsstoffe, Gustav Fischer Verlag - Stuttgart-New York - 1980, S. 55, 56.

Völlig überraschend hat sich nun gezeigt, daß erfindungsgemäß die Ester von Terpenen (Terpenalkoholen) mit $\alpha$-Ketocarbonsäuren und/oder Kombination aus einem oder mehreren Terpenen zusammen mit einer oder mehreren $\alpha$-Ketocarbonsäuren einen synergistischen Effekt zeigen. Die Terpene können auch, wenigstens teilweise, in Form ihrer Ester oder Äther, die Säuren können z.B. teilweise, auch in Form ihrer Ester und Salze vorliegen. Als Terpene kommen, soweit in den erfindungsgemäßen bioaktiven Mitteln Terpen-ester, also Terpenylester, enthalten sind, Terpenalkohole in Betracht, insbesondere Alkohole, denen der Strukturtyp des Bornans und Menthans einschließlich ihrer Derivate, zugrundeliegt. Beispiele hierfür sind Borneol, Isoborneol, Menthol und dergleichen. Es sind aber als Terpene auch Verbindungen vom Strukturtyp des Pinans, Carans und Thujans, einschließlich ihrer Derivate, deren Alkohole, z.B. $\alpha$-Pinen, ß-Pinen, $\Delta^3$-Caren ohne weiteres verwendbar. Monoterpene sind bevorzugt. Namentlich dann, wenn, wie in den Wirkstoff-Kombinationen b) ein Terpenester nicht erforderlich ist, können Terpene beliebiger Art, also Terpene aus der ganzen Terpen-Stoffgruppe, einschließlich Terpen-Gemischen, enthalten sein. Soweit in den Wirkstoff-Kombinationen veresterte Terpene enthalten sind, handelt es sich hierbei bevorzugt um Ester niedriger Alkansäuren oder auch höherer Homologen hiervon mit den Terpenalkoholen. Beispiele sind die Formiate, Acetate, Propionate und Isopropionate. Sind die in den Wirkstoff-Kombinationen enthaltenen Terpene z.B. zum Teil veräthert, so handelt es sich vorzugsweise um Äther mit niedrigen Alkanolen. Die $\alpha$-Ketocarbonsäuren sind bevorzugt niedrige $\alpha$-Ketomono- und dicarbonsäuren, insbesondere Glyoxylsäure, Brenztraubensäure, Oxalbernsteinsäure. Soweit in Wirkstoffkombinationen Ester und/ oder Salze der $\alpha$-Ketocarbonsäuren enthalten sind, handelt es sich hierbei bevorzugt um Ester mit niedrigen Alkanolen z.B. um Äthyl-ester und dergl. Ester der $\alpha$-Ketocarbonsäure mit pharmakologisch unbedenklichen Alkoholen. Von den Salzen sind Alkalimetallsalze und Erdalkalimetallsalze, z.B. Calciumsalze, bevorzugt.

Mit den erfindungsgemäßen Mitteln treten die bioaktiven Wirkungen wesentlich stärker als bei Terpen bzw. den Terpenen allein auf. Dies hat sich bevorzugt gezeigt an $\alpha$-Ketocarbonsäuremonoterpenylestern, wie 1-Bornylglyoxylat, 1-Isobornylglyoxylat, 1-Mentyhlglyoxylat, 1-Bornylpyruvat, 1-Isobornylpyruvat, 1-Menthylpyruvat bzw. an Monoterpen-$\alpha$-Ketocarbonsäure-Kombinationen von 1-Borneol, 1-Bornen, 1-Isoborneol, 1-Bornylacetat, 1-Isobornylacetat, 1-Menthol und/oder 1-Menthylacetat mit Glyoxylsäure, Glyoxylsäureäthylester, Brenztraubensäure und/oder Brenztraubensäureäthylestern.

Es macht dabei keinen Unterschied, ob $\alpha$-Ketomonocarbonsäuren oder $\alpha$-Ketodicarbonsäuren wie Oxalessigsäure, Oxalbernsteinsäure, $\alpha$-Ketoglutarsäure, Zwischenprodukte des Zitronensäurezyklus und deren Mono- wie Diester (auch gemischte Ester) verwendet werden. So zeigen auch die $\alpha$-Ketodicarbonsäuremonoterpenylmonoester und $\alpha$-Ketodicarbonsäuremonoterpenyldiester bzw. die obigen Dicarbonsäuren, ihre Ester, wie die Monoäthyl- oder Diäthylester in Kombinationen mit den Monoterpenen verstärkte bioaktive Wirkungen. Beide Stoffklassen Terpene und $\alpha$-Ketocarbonsäuren, besonders Monoterpene, vorzugsweise die eben aufgeführten, wie 1-Borneol 1-Isoborneol, 1-Bornylacetat, 1-Isobornylacetat, 1-Menthol, 1-Mentylacetat, und die beiden untersten Vertreter der homologen Reihe der $\alpha$-Ketocarbonsäuren bzw. $\alpha$-Ketocarbonsäureester, Glyoxylsäure, Glyoxylsäureäthylester, Brenztraubensäure, Brenztraubensäureäthylester umfassen wichtige natürliche Substanzen des Stoffwechsels sowohl im Tier- wie auch im Pflanzenreich. Kombinationen dieser Substanzen bzw. die entsprechenden $\alpha$-Ketocarbonsäureterpenylester, bevorzugt Glyoxylsäuremonoterpenyl- und Brenztraubensäuremonoterpenylester (Monoterpenalkohole siehe oben) lassen ein geringeres Toxizitätsrisiko erwarten als rein synthetische Substanzen.

Die bioaktiven Wirkungen stellen sich nicht nur temporär, sondern langanhaltend profund ein und deuten auf eine regenerative Wirkung hin.

Die bioaktiven Wirkungen treten gegen Beschwerden des rheumatischen Formenkreises, Arthriden, Arthrose, Gicht, Gichtknoten, Erkrankungen des Bewegungsapparates, deformative Veränderungen der Bandscheiben und Gelenke, Sehnenscheidenentzündung, Knochenhautentzündung, Muskelverspannungen, Muskelkater, Prellungen, Schwellungen, Insektenstich, Hühneraugen, Furunkel, Ausschläge, Akne, Pickel, Exzeme, Hautentzündungen, Allergien, Hautflechte, Psoriasis (Schuppenflechte), Verbrennungen, Sonnenbrand, Überbein, Wundheilprozesse, Hämorrhoiden u.a. ein.

Bemerkenswert ist, daß diese Wirkungen nur von Corticoiden erreicht werden, daß es sich aber bei den genannten bioaktiven Mitteln keineswegs um "Corticoide Systeme" handelt. Die Wirkstoffkombinationen bzw. Wirkstoffe stellen somit eine echte Alternative gegen die mit sehr starken Nebenwirkungen behafteten "Corticoiden Systeme" dar.

Als Terpenquelle z.B. auch für l-Borneol, l-Bornylacetat, l-Lornylformiat und deren Gemische können (z.B. äthanolische) Extrakte aus Fichten-, Tannen-, Latschenkiefernadeln verwendet werden, aber auch andere natürliche Terpenquellen wie die Pflanzenöle Rosenöl, Pfefferminzöl, Campheröl, Fieberkleeöl, Lindenblütenöl, Wermutöl, Fischleberöle, Olivenöl, Lavendelöl, Kümmelöl, Zitronenöl, Lemongrasöl, Anisöl, Nelkenöl, Fenchelöl, Wacholderöl, Eucalyptusöl, Zimtöl, Ingweröl, Gelbwurzöl, Kardamonenöl, Terpentinöl, Bukkoblätteröl, Pomeranzenschalenöl, Angelikawurzelöl, Liebstöckelöl, Melissenöl, Salbeiöl, Thymianöl, Quendelöl, Baldrianwurzelöl, Arnikeblütenöl, Kamillenblütenöl, Schafgarbenöl, Lärchennadelöl, Bartgrasöl u.a.. können verwendet werden.

Es können also, sowohl zusammen mit den $\alpha$-Ketocarbonsäureterpenylestern, als auch in den Terpen-$\alpha$-ketocarbonsäure-Wirkstoff-Kombinationen, Gemische von unterschiedlichen Terpenen, z.B. Bornen-haltige Gemische wie l-Borneol, Bornen, Ester und/oder Äther des Borneols usw. verwendet werden. So können bevorzugt auch natürliche Terpengemische, wie z.B. Extrakte aus den obengenannten natürlichen Stoffen im Rahmen der Erfindung als Terpene enthalten sein.

Die erfindungsgemäßen bioaktiven Mittel können die üblichen Zusatzstoffe, wie Stabilisatoren, Streck- und Verdünnungsmittel, Trägermedien, wie pflanzliche Öle und Fette und Wachse, Neutralöl, Äthanol, Isopropanol, enthalten.

Zur Erläuterung der chemischen Begriffe:

Verestert mit Essigsäure    &rarr;Bornylacetat

Isobornylacetat

veräthert mit Methanol, Äthanol→Bornyl-,Isoborny.

äther

Borneol

Isoborneol

Bornen                  Menthol                  Menthylacetat

Bornylglyoxylat                          Bornylpyruvat

Isobornylglyoxylat                    Isobornylpyruvat

Menthylglyoxylat                        Menthylpyruvat

Die nun folgenden Beispiele dienen der Erläuterung der Erfindung: Die angegebenen %-Zahlen sind Gew.-%, sofern nicht anderes bemerkt ist. Die einzelnen Stoffe können dabei in beliebiger Reihenfolge gemischt werden.

Medizinische Salben und Öle

Beispiel 1

1%     1-Bornylacetat (oder 1 % 1-Borneol)

1%     Brenztraubensäureäthylester (oder 1% Brenztraubensäure)

97,5% Salbengrundlage (fettend oder nicht fettend)

0,2%   Ascorbinsäure

0,3%   Zitronensäure

Beispiel 2

1%     Bornylacetat (oder 1% 1-Borneol)

1%     Brenztraubensäureäthylester (oder 0,5% Calciumpyruvat)

97%     Salbengrundlage (fettend oder nicht fettend)

0,2%   Ascorbinsäure

0,8%   Zitronensäure

oder auch 1% Brenztraubensäure, dafür aber 0,8% Calciumcitrat

Beispiel 3

1%     . 1-Bornylacetat (oder 1% 1-Borneol)

1%     Brenztraubensäureäthylester (oder 1% Brenztraubensäure)

98%     Neutralöl (oder Oliven-, Sonnenblumenöl ect.)

- 8 -

### Beispiel 7

| 1,5% | 1-Menthylpyruvat | (oder Mischung 0,75% 1-Menthol |
| 98,5% | Salbengrundlage | oder 1-Menthylacetat und 0,75% |

Glyoxylsäure oder Glyoxylsäureäthylester)

### Beispiel 8

| 1,5% | 1-Bornylglyoxylat | (oder Mischung 0,75% 1-Borneol |
| 98,5% | Sonnenblumen- oder | oder 1-Bornylacetat und 0,75% |
| | Neutralöl oder | Glyoxylsäure oder Glyoxylsäure- |
| | Salbengrundlage | äthylester) |

### Beispiel 9

| 2% | 1-Isobornylglyoxylat | (oder Mischung 1% 1-Isoborneol |
| 97,5% | Salbengrundlage | oder 1-Isobornylacetat und |
| 0,5% | Zitronensäure | 1% Glyoxylsäure oder Glyoxyl- |

säureäthylester oder 1% Calciumglyoxylat)

### Beispiel 10

| 2% | d,1-Methylglyoxylat | (oder Mischung 1% d,1-Menthol oder |
| 98% | Neutralöl oder Iso- | d,1-Menthylacetat und 1% Glyoxyl- |
| | propanol | säure oder Glyocylsäureäthylester |

Beispiel 4

1%    1-Bornylacetat   (oder 1% 1-Borneol)

1%    Brenztraubensäureäthylester (oder 1% Brenztraubensäure)

98%   Isopropanol

Diese alkoholische Lösung kann auch bevorzugt als Mittel gegen Haarausfall oder ins Badewasser als Rheumabad oder Erfrischungsbad (bei Sportlern, auch gegen Muskelkater) verwendet werden.

Beispiel 5

In den Beispielen 1 - 4 anstelle von Bornylacetat 1-Isobornylacetat (oder 1-Isoborneol) gesetzt.

Beispiel 6

In den Beispielen 1 bis 5 können für 1-Bornylacetat, 1-Isobornylacetat, 1-Borneol, 1-Isoborneol auch die Ester wie 1-Bornylpyruvat bzw. 1-Isobornylpyruvat eingesetzt werden:

2%    1-Bornylpyruvat   (oder 2% Isobornylpyruvat)

98%   Neutralöl oder Sonnenblumenöl oder Isopropanol

Beispiel 11

1%     Bornen, Borneoläthyläther, Bornylacetat (1:1:1)

1%     Brenztraubensäureäthylester

98%    Salbengrundlage


Beispiel 12

2%     Oxalessigsäure-1-1-bornylester       (oder Mischung 1% Oxalessi

98%    Salbengrundlage                      säure oder Oxalessigsäure-

                                            1-äthylester und 1% 1-Bor-

                                            neol oder 1-Bornylacetat)


Beispiel 13

2%     Oxalessigsäure-1,4-di-1-             (oder Mischung 1% Oxal-

       bornylester                          essigsäure oder Oxalessig-

98%    Neutralöl oder Salbengrundlage       säure-1-äthylester oder

                                            Oxalessigsäure-1,4-diäthyl

                                            ester und 1% 1-Borneol od.

                                            1-Bornylacetat)


Beispiel 14

2%     Oxalessigsäure-1-1-bornyl-4-         (oder Mischung analog 12,

       äthyldiester                         13)

98%    Salbengrundlage

Unter Oxalessigsäure-1-l-bornylester ist

l-Bornyl-OOC-CO-CH$_2$-COOH mit numerierten C-Atomen zu verstehen.
$\quad\quad\quad$ 1 2 $\quad$ 3 $\quad\quad$ 4

Für kosmetische Salben werden die gleichen Rezepturen mit geringerer Wirkstoffkombinations- bzw. Wirkstoffdosierung angewendet.

- 1 -

<u>Patentansprüche :</u>

1. Bioaktives, Terpen-Verbindungen enthaltendes Mittel, insbesondere Arzneimittel und kosmetische Mittel, g e - k e n n z e i c h n e t   d u r c h   einen Gehalt an

a)  $\alpha$-Ketocarbonsäureterpenylester und/oder

b)  einer Kombination von Terpen mit $\alpha$-Ketocarbonsäure.

2. Bioaktives Mittel gemäß Patentanspruch 1,  d a - d u r c h   g e k e n n z e i c h n e t ,  daß die $\alpha$-Ketocarbonsäure eine $\alpha$-Ketomonocarbonsäure und/oder eine $\alpha$-Ketodicarbonsäure, vorzugsweise Glyoxylsäure, Brenztraubensäure, Oxalessigsäure, Oxalbernsteinsäure und/oder $\alpha$-Ketoglutarsäure ist.

3. Bioaktives Mittel gemäß Patentanspruch 1 oder 2, d a d u r c h   g e k e n n z e i c h n e t ,  daß in der Kombination b) die $\alpha$-Ketocarbonsäure, zumindest teilweise, in Form ihrer Ester oder als Estergemische, ins-

- 2 -

besondere Mono- oder Diäthylester, oder in Form ihrer Salze, insbesondere Alkali- oder Erdalkalimetallsalze, enthalten ist.

4. Bioaktives Mittel gemäß einem der vorhergehenden Patentansprüche, d a d u r c h  g e k e n n z e i c h - n e t , daß dem Terpen ein Terpenalkohol zugrunde liegt, insbesondere Alkohole, denen der Strukturtyp des Bornans, Menthans oder denen Derivate dieser Strukturtypen zugrundeliegen.

5. Bioaktives Mittel gemäß einem der vorhergehenden Patentansprüche, d a d u r c h  g e k e n n z e i c h - n e t , daß dem Terpen Borneol, Isoborneol und/oder Menthol in der d-, l- oder dl-Form, vorzugsweise in der l-Form, zugrundeliegt.

6. Bioaktives Mittel gemäß einem der vorhergehenden Patentansprüche, d a d u r c h  g e k e n n z e i c h - n e t , daß in der Kombination b) das Terpen als freie Verbindung, als Ester und/oder als Äther, als Ester vorzugsweise mit pharmakologisch unbedenklichen niedrigen Alkansäuren, als Äther vorzugsweise von niedrigen Alkano-len, insbesondere als l-Bornylacetat, l-Isobornylacetat, l-Menthylacetat oder als Methyl- oder Äthyläther von l-Borneol, l-Isoborneol oder l-Menthol oder als Terpen-Gemisch vorliegt.

7. Bioaktives Mittel gemäß einem der vorhergehenden Patentansprüche, d a d u r c h   g e k e n n z e i c h n e t , daß in der Kombination b) als Ester, gegebenenfalls zusätzlich, ein $\alpha$-Ketocarbonsäureterpenylester enthalten ist.

8. Bioaktives Mittel gemäß einem der vorhergehenden Patentansprüche, d a d u r c h   g e k e n n z e i c h n e t , daß die Ester Bornylglyoxalat, Isobornylglyoxalat, Menthylglyoxalat, Bornylpyruvat, Isobornylpyruvat und/oder Menthylpyruvat, vorzugsweise in der l-Form, sind.

9. Bioaktives Mittel gemäß einem der vorhergehenden Patentansprüche, d a d u r c h   g e k e n n z e i c h n e t , daß die Terpene aus natürlichen Terpenquellen, vorzugsweise aus Fichten-, Tannen- und/oder Latschenkiefernadeln, gewonnen durch Extraktion, stammen.

10. Bioaktives Mittel gemäß einem der vorhergehenden Patentansprüche, d a d u r c h   g e k e n n z e i c h n e t , daß Terpengemische, insbesondere Bornen, Borneolalkyläther und/oder Borneolalkylester umfassende Gemische enthalten sind.

11. Verwendung der bioaktiven Mittel gemäß einem der vorhergehenden Patentansprüche zur äußerlichen oder parenteralen Anwendung.

# Europäisches Patentamt
# EUROPÄISCHER RECHERCHENBERICHT

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl. 3) |
|---|---|---|---|
| A | DE-A-2 436 468 (HENKEL & CIE GmbH) | 1-11 | A 61 K 31/215 |
| | | | A 61 K 31/19 |
| | --- | | A 61 K 7/48 |
| A | US-A-3 920 835 (EUGENE J. VAN SCOTT) | 1-11 | A 61 K 7/06 // (A 61 K 31/19 A 61 K 31/045) |
| | --- | | |
| A | FR-M- 2 940 (LABORATOIRES HOUDE S.A.) | 1-11 | |
| | --- | | |
| A | FR-A-2 085 627 (LABORATOIRES VALDA) | 1-11 | |
| | ----- | | |

RECHERCHIERTE
SACHGEBIETE (Int. Cl. 3)

A 61 K
C 07 C

Der vorliegende Recherchenbericht wurde für alle Patentansprüche erstellt.

| Recherchenort DEN HAAG | Abschlußdatum der Recherche 21-06-1982 | Prüfer BRINKMANN C. |
|---|---|---|